# EUROPEAN PATENT SPECIFICATION

(11) **EP 1 176 149 B1**
(45) Date of publication and mention of the grant of the patent: **06.08.2003**
(21) Application number: 00901035.6
(22) Date of filing: 21.01.2000
(51) Int. Cl.: C07H 15/256, C07J 63/00, A61K 31/70, A61K 35/78

(54) **NOVEL GYMNEMIC ACID DERIVATIVES, PROCESS FOR THE PREPARATION THEREOF AND USE THEREOF AS MEDICINE**
DERIVATE DER GYMNEMISCHEN SÄURE, VERFAHREN ZU IHRER HERSTELLUNG UND IHRE VERWENDUNG ALS MEDIKAMENTE
NOUVEAUX DERIVES DE L'ACIDE GYMNEMIQUE, LEUR PROCEDE DE PREPARATION ET LEUR APPLICATION COMME MEDICAMENTS

(30) Priority: 11.02.1999 CN 99100721; 11.02.1999 CN 99100722; 12.03.1999 CN 99102823; 05.04.1999 CN 99103588
(43) Date of publication of application: 30.01.2002
(73) Proprietor: Guilin Jiqi Pharmaceutical Co., Ltd., Guangxi 541004 (CN)
(72) Inventor: YE, Wencai China Pharmaceu. Uni. Phytochem. Dept., Nanjing Jiaangsu Province 210038 (CN); DAI, Yue China Pharmaceu. Uni. Pharmacology Dept., Nanjing Jiangsu Province 210038 (CN); CONG, Xiaodong, Nanjing Jiangsu Province 210009 (CN); ZHU, Xingxiang, Nanjing Jiangsu Province 2100009 (CN); ZHAO, Shouxun, Nanjing Jiangsu Province 2100009 (CN)
(74) Representative: Ricker, Mathias, Dr. Dipl.-Chem.
(86) International application number: PCT/CN00/00010
(87) International publication number: WO 00/047594

(56) References cited:
- EP-A1- 0 406 516
- US-A- 5 843 909
- YOSHIKAWA M ET AL: "BIOACTIVE SAPONINS AND GLYCOSIDES VII. ON THE HYPOGLYCEMIC PRINCIPLES FROM THE ROOT CORTEX OF ARALIA ETALA SEEM.: STRUCTURE RELATED HYPOGLYCEMIC ACTIVITY OF OLEANOLIC ACID OLIGOGLYCOSIDE" CHEMICAL AND PHARMACEUTICAL BULLETIN, PHARMACEUTICAL SOCIETY OF JAPAN. TOKYO, JP, vol. 44, no. 10, October 1996 (1996-10), pages 1923-1927, XP001061640 ISSN: 0009-2363
- YOSHIKAWA M ET AL: "MEDICINAL FOODSTUFFS. X. STRUCTURES OF NEW TRITERPENE GLYCOSIDES, GYMNEMOSIDES-C, -E, AND -F, FROM THE LEAVES OF GYMNEMA SYLVESTRE R. BR.: INFLUENCE OF GYMNEMA GLYCOSIDES ON GLUCOSE UPTAKE IN RAT SMALL INTESTINAL FRAGMENTS" CHEMICAL AND PHARMACEUTICAL BULLETIN, PHARMACEUTICAL SOCIETY OF JAPAN. TOKYO, JP, vol. 45, no. 12, December 1997 (1997-12), pages 2034-2038, XP001061735 ISSN: 0009-2363
- YOSHIKAWA M ET AL: "MEDICINAL FOODSTUFFS VII. ON THE SAPONIN CONSTITUENTS WITH GLUCOSE AND ALCOHOL ABSORPTION-INHIBITORY ACTIVITY FROM A FOOD GARNISH TONBURI, THE FRUIT OF JAPANESE KOCHIA SCOPARIA (L.) SCHRAD.: STRUCTURES OF SCOPARIANOSIDES A, B, AND C" CHEMICAL AND PHARMACEUTICAL BULLETIN, PHARMACEUTICAL SOCIETY OF JAPAN. TOKYO, JP, vol. 45, no. 8, 1997, pages 1300-1305, XP001061675 ISSN: 0009-2363
- PATENT ABSTRACTS OF JAPAN vol. 1996, no. 06, 28 June 1996 (1996-06-28) & JP 08 040912 A (RES INST FOR PROD DEV), 13 February 1996 (1996-02-13)
- MASUDA, HIROYUKI ET AL: "Intraperitoneal administration of Senegae Radix extract and its main component, senegin-II, affects lipid metabolism in normal and hyperlipidemic mice" BIOL. PHARM. BULL. (1996), 19(2), 315-17 , 1996, XP001063179
- TAN N ET AL: "Advances in structural elucidation of glucuronide oleanane-type triterpene carboxylic acid 3,28-O-bisdesmosides (1962-1997)" PHYTOCHEMISTRY, PERGAMON PRESS, GB, vol. 52, no. 2, September 1999 (1999-09), pages 153-192, XP004290983 ISSN: 0031-9422
- CHEM. PHARM. BULL., vol. 37, no. 3, 1989, pages 852 - 854
- TETRAHEDRON LETTERS, vol. 30, no. 12, 1989, pages 1547 - 1550

## Description

### The Invention Field

This invention relates to new Gymnemic acid derivatives, their preparation, pharmaceutical composition or extract which contains them, and their medical use, especially the use in the prevention or treatment of the diseases associated with hyperglycemia, hyperlipidemia and platelets aggregation.

### Background of technology

A lot of study on Gymnemic Acid derivatives have been done and all of these Gymnemic acid derivatives are from the plant called Gymnema cane, which is classified as Gymnema sylvestre. R. Br. And in India, it has been used to treat swelling, snake venom toxin, malaria, as a diuretic or to lower blood sugar lever (see Yoshikawa et al., Chem. Pharm. Bull 44/10), 1923-27 (1996)). Yet the Gymnemic acid derivatives and their biological activity mentioned in this invention haven't been reported up to date. Other Gymnemic acid derivatives also show anti-hyperlipidemic activities (see Masuda et al., Biol. Pharm. Bull., 19(2), 315-317 (1996)).

### The object of this invention

The object of this invention is to find new Gymnemic acid derivatives and develop their medical use.

### Description of this invention

This inventor has found out new Gymnemic acid derivatives formula I or II and further their medical use, especially in treating hyperglycemia, hyperlipidemia and platelets aggregation. The invention is now performed based on the discovery mentioned above.

In the first part, this invention concerns Gymnemic Acid derivatives formula I or II, 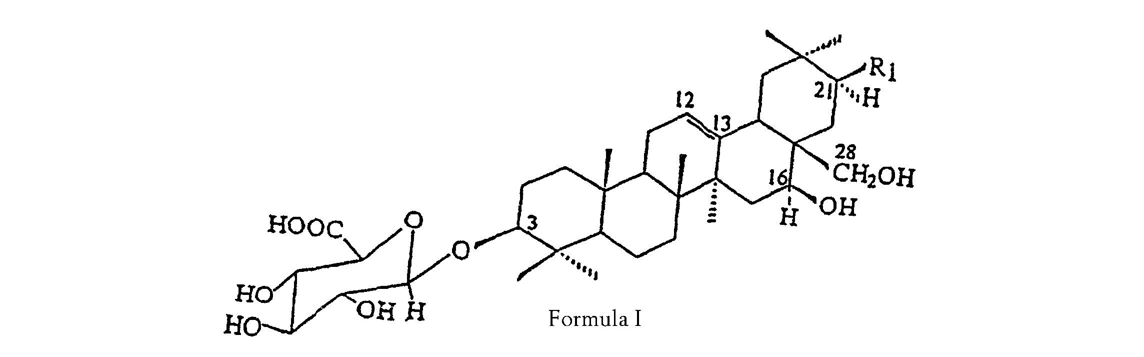 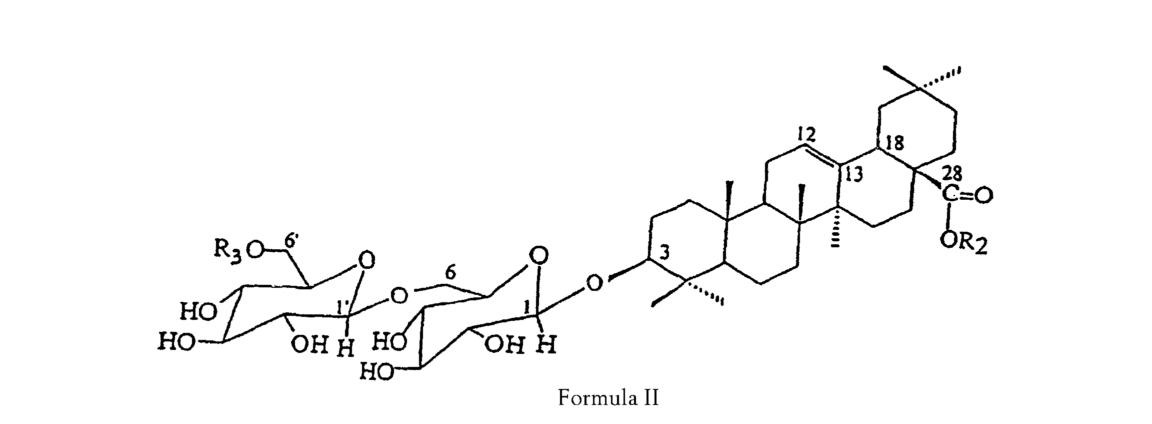 wherein, R₁ is H or the radical represented by the following formula 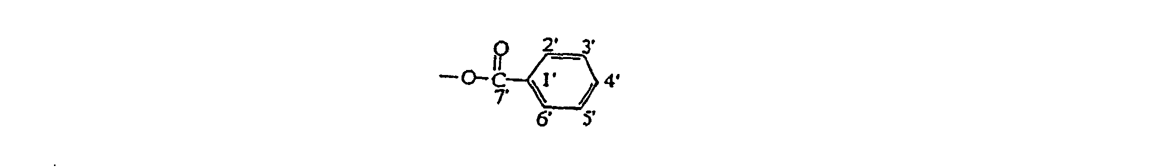 R₃ is H, and R₂ symbolizes the following radical, or 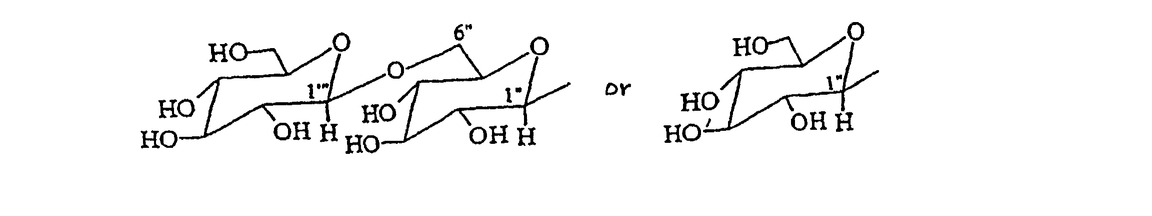 R₃ symbolizes the following radical, 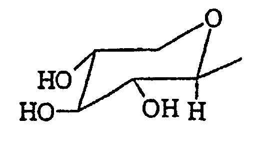 R₂ is H or the following radical, 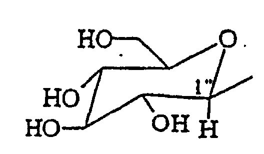 or pharmaceutically base addition salt thereof.

The second part of this invention relates to pharmaceutical composition which contains at least one kind of Gymnemic Acid derivative of formula I and/or II or pharmaceutical base addition salt thereof as active ingredient, pharmaceutical carrier and excipient.

The third part of the invention involves Gymnemic Acid extract, 12.5-40wt% of which is Gymnemic Acid derivative of formula I and/or II.

Another part of this invention relates to pharmaceutical composition for the prevention or treatment of the diseases associated with hyperglycemia, hyperlipidemia and platelets aggregation, which contains at least one kind of Gymnemic Acid derivative of formula I and/or II or pharmaceutical base addition salt thereof as a active ingredient, pharmaceutical carrier and excipient.

Another part of the invention relates to a pharmaceutical composition for the prevention or treatment of diabetic, which includes at least one kind of Gymnemic Acid derivative of formula I and/or II or pharmaceutical base addition salt thereof as a active ingredient, officinal carrier and excipient.

Another part of this invention relates to pharmaceutical composition for the prevention or treatment of higher blood lipid level, which contains at least one kind of Gymnemic Acid derivative of formula I and/or II or pharmaceutical base addition salt thereof as a active ingredient pharmaceutical carrier and excipient.

Another part of this invention relates to pharmaceutical composition for the prevention or treatment of platelets aggregation, which contains at least one kind of Gymnemic Acid derivative of formula I and/or II or pharmaceutical base addition salt thereof as a active ingredient, pharmaceutical carrier and excipient.

Another part of this invention relates to the preparation of Gymnemic Acid derivative of formula I and II or pharmaceutical base addition salt thereof, which includes the following steps:
a) extracting the plant Gymnema cane with ethanol under reflux and then concentrating;
b) extracting concentrated liquid in step a) with cyclohexane , then extracting with n-butanol, concentrating to dryness under reduced pressure, and then obtaining a ointment;
c) subjecting the ointment in step b) to silica column chromatography with elute as chloroform: methanol=90:10-50:5 or 90:10-60:40, obtaining Gymnemic acid derivative of formula I and residue;
d) subjecting the residue in step c) to C₁₈ column chromatography with elute as methal/water (20/80-40/60), obtaining Gymnemic acid derivative of formula II;
e) if desired, converting the obtained Gymnemic acid derivative of formula I or II into pharmaceutical base addition salt with inorganic or organic base.

Another part of this invention relates to a method of preparation of the extract containing Gymnemic Acid derivative of formula I and II which range from 12.5-40wt%, which includes the following steps:
a)extracting Gymnema cane leaves with 60-95% ethanol and concentrating,
b)extracting concentrated liquid in step a) with cyclohexane, then extracting with n-butanol, and then concentrating the extract under reduced pressure.

Another aspect of the invention relates to use of Gymnemic Acid derivative of formula I and II or the extract containing Gymnemic Acid derivative of formula I and II for the manufacture of medicament for the prevention or treatment of the diseases and conditions associated with hyperglycemia, hyperlipidemia and platelets aggregation.

Finally, this invention relates to the method of preventing or treating the diseases and conditions associated with hyperglycemia, hyperlipidemia and platelets aggregation, which includes administrating prophylactic or treatment effective quantity of Gymnemic Acid derivative of formula I and II to the patient suffered from diseases or conditions associated with hyperglycemia, hyperlipidemia and platelets aggregation.

The term "patient" in the invention refers to mammal, including human being, and especially human being.

### Detailed Description of the Invention

This invention relates to Gymnemic Acid derivative of formula I and II, 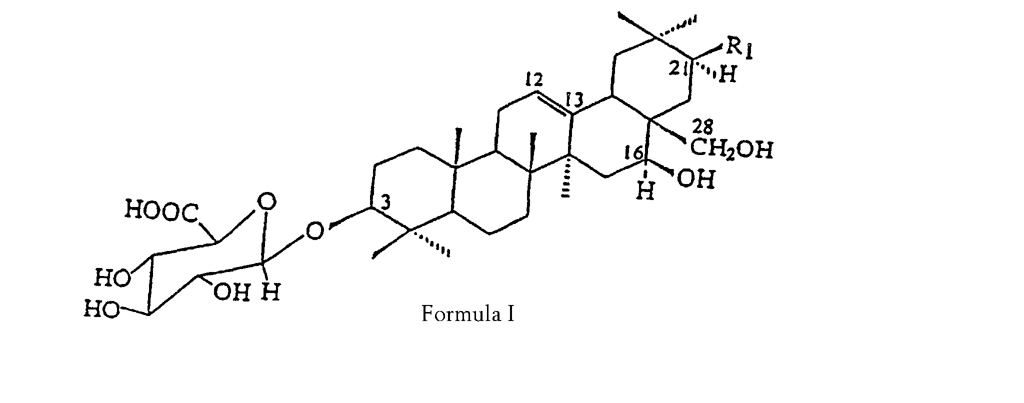 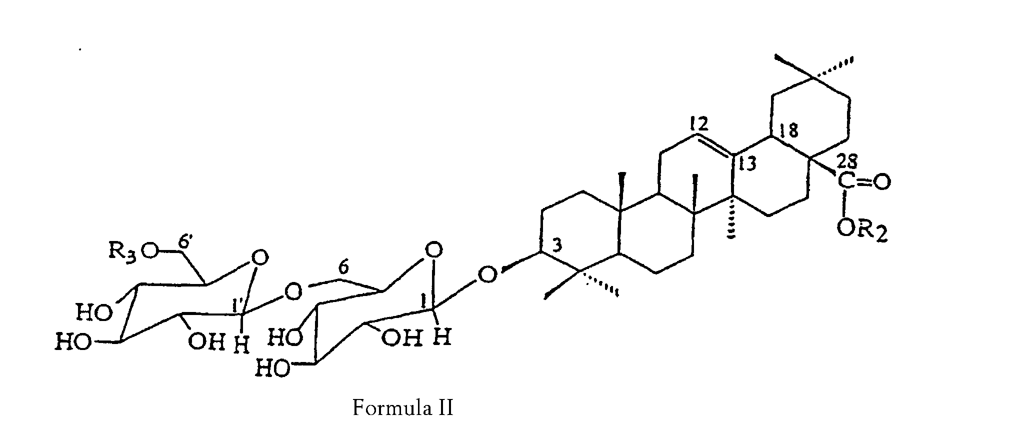 Wherein, R₁ is H or the group of the following formula 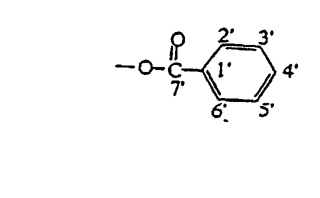 R₃ is H, R₂ is the following group, or 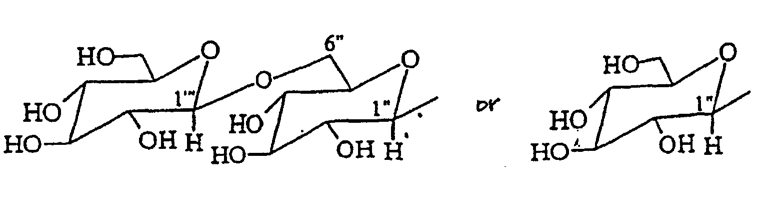 R₃ is the following group, 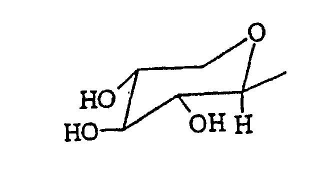 R₂ is H or the following group, 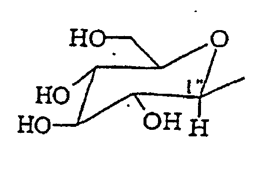 or pharmaceutical base addition salt.
According to the present invention, pharmaceutical base addition salt of Gymnemic acid of formula I or II includes a salt formed with pharmaceutical inorganic or organic base, inorganic base, for example, includes alkali or alkali earth metal hydroxide, alkali metal or alkali earth metal carbonate or bicarbonate, alkali metal may be selected from Li, Na, K, alkali earth metal may be selected from Ba, Mg, Ca etc. The organic base, for example, may be triethyl amine etc.

According to this invention, Gymnemic acid compound prefers Gymnemic Acid compound of formula I wherein R₁ is H..

According to the present invention, Gymnemic acid compound prefers Gymnemic Acid compound of formula I wherein R₁ as the following radical. 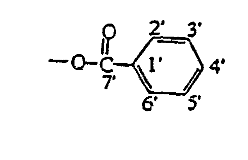 According to the present invention, Gymnemic acid compound prefers Gymnemic Acid compound of formula II wherein R₃ as H and R₂ as the following radical. 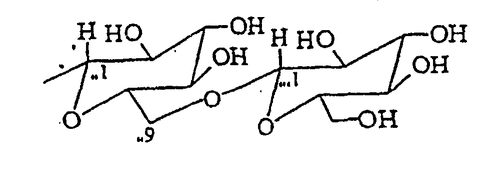 According to the present invention, Gymnemic acid compound prefers Gymnemic Acid compound of formula II wherein R₃ as H and R₂ as the following radical. 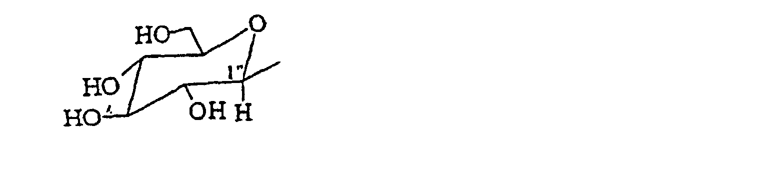 According to the present invention, Gymnemic acid compound prefers Gymnemic Acid compound of formula II wherein R₃ as the following radical and R₂ as H. 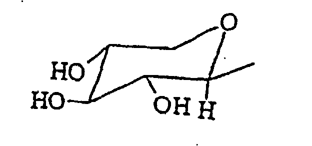 According to the present invention, Gymnemic acid compound prefers Gymnemic Acid compound of formula II wherein both R₃ and R₂ as the following radical respectively.
R₂ is 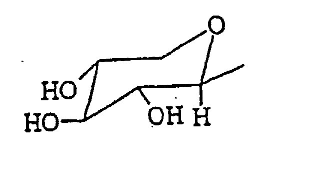 R₃ is 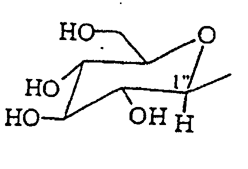 According to this invention, the pharmaceutical composition mentioned here contains at least one kind of Gymnemic Acid derivative of formula I and/or II and pharmaceutical carrier and excipient. For example, the pharmaceutical composition may include, for example, 1.25-2.10wt% compound A, 0.89-1.50wt% compound B, 2.40-3.80wt% compound C, 2.10-3.40wt% compound D, 2.74-4.60wt% compound E, and 3.24-5.40wt% compound F (compounds A, B, C, D, E and F as defined in examples below.). This pharmaceutical composition can be administrated by gastro intestine, parenteral or topical administration, such as oral, muscle, subcutaneous, peritonaeum, vein etc. The forms of drug suitable for intestine administration are for example tablet, capsule, solution, suspension, powder, granulate etc. The forms of drug suitable for parenteral include injection solution, frozen dry powder for injection etc. The drug forms suitable for the topical are for example, ointment, cream, paste, patch, and spray. Of all these forms, oral administration is preferred while capsule is preferred in oral form. The pharmaceutical carrier or excipient of the pharmaceutical composition includes binding agent, filling material, wet agent, desintegrate agent , surfactant, lubricating agent ,dilut agent etc. If desired, colour agent, flavoring agent, solubilizer, buffer etc are also used. The diluting agents in the invention include starch, dextrin, lactose, microcellulose, silica gel, etc. And silica gel is preferred. The wetting agents includes water and ethanol, lubricating agents include talcum powder, stearic magnesium.

The pharmaceutical composition in the present invention can be produced by the known method in this art. For example, mix Gymnemic Acid derivative of formula I and/or II or pharmaceutical base addition salt with pharmaceutical carrier and excipient.

The dose of Gymnemic Acid derivative of formula I and II depends on many factors such as the characters and seriousness level of the disease to be prevented or treated, sex, age, weight, individual response, specific compound, administration route and times of administration. Generally the specific dose depends on the judgment of physician. Generally speaking, the dosage the pharmaceutical composition Gymnemic Acid derivative of formula I and II can be in the form of single dose and taken 1-4 times per day.

According to this invention, the or pharmaceutical base said of formula I Gymnemic Acid derivative can be prepared as follows:
a) crushing dry leaves of Gymnema cane, then extracting three times with 60-95% ethanol under reflux, two hours for each, combining extracted liquid and concentrating under reduced pressure until there was no ethanol, for use;
b) extracting the concentrated mixtures in step a) for 3 to 6 times with cyclohexane , then extracting with n-butanol, concentrating to dryness under reduced pressure, obtaining dry extract, ready for use;
c) subjecting the dry extracts in step b) to silica gel column chromatography with elute as mixture of chloroform and methanol at the ratio 90:10 to 60:40, obtaining derivatives of formula I,
d) If desined, converting the derivative of formula I in step c) into pharmaceutical base salt thereof.
According to this invention, the Gymnemic Acid derivative of formula II can be prepared as follows:
a) Crushing dry leaves of Gymnema cane, then extracting three times with 60-95% ethanol under reflux, two hours for each, combining extracted liquid and concentrating under reduced pressure until there was no ethanol, ready for use.
b) extracting concentrated mixtures for 3 to 6 times with cyclohexane , then extracting with n-butanol; concentrating to dryness under reduced pressure, ready for use;
c) mixing the dry extracts in step b) with raw silica gel; subjecting separation with thin layer chromatography of silica gel H; the mixture of chloroform and methanol at the ratio 90:10 to 50:50 as elute, subjecting the residue after elute to C₁₈ column chromatography with elute as methanol water (20:80-40:60), obtaining derivative of formula II;
d) if desired, converting the derivative of formula II in step c) into pharmaceutical base salt therof.
According to this invention, the extract products with 12.5-40 wt% Gymnemic Acid derivative of formula I and formula II can be prepared as follows: raw powder of Gymnema cane leaves were refluxed 1-4 times with 60-95% ethanol, the amount of solvent for each is 6ml/g, extract time is 1-3 hours. The extract mixtures were combined together and distilled under reduced pressure till there was no ethanol, the concentrated mixture was extracted with cyclohexane for 1-3 times, 500ml solvents was used for each time. Then the mixture was extracted for 1-3 times 500ml with n-butanol, all the extract mixtures were combined and distilled under reduced pressure to obtain the desired product.

This invention gives a further illustration by the preparation examples and biological active experiment, but it does not means any limitation to the invention.

### Example 1

Preparation of compound A (Gymnemic Acid derivative of formula I wherein the R₁ being H) and compound B (Gymnemic Acid derivative of formula I wherein the R₁ being group as follow)

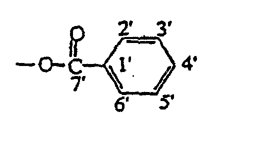

1000g raw powder of Gymnema cane leaves were refluxed for 3 times with 60% ethanol, 6L solvents were used for each, and 2 hours for each time. The extract mixtures were combined together and distilled under reduced pressure till there was no ethanol, the concentrated mixture was extracted with 0.5L cyclohexane and butane for 3 times. All the n-butane extract mixtures were combined and distilled under reduced pressure to obtain 64.0g dry extract product. 32.0g dry extract substance was added into 60g 60-100 mesh rough silica gel, the mixture was vaporized to dryness on a water pan, 450g 200-300 mesh (m) silica gel were loaded into column by a wet method, then treated sample was added to be subjected to column separation with elute as 90:10-60:40 of mixtures of chloroform and methanol mixtures ,80mg compound A and 60mg compound B were obtained.

The physics and chemistry data of compound A and compound B were showed as follows:

### Compound A:

Amorphous powder: mp198 - 202°C; [α]₂₀^{D}+16.0° ( c0.10, MeOH ); IRνₘₐₓ3414 ( OH ), 1724 ( COOH ), 1636 (C=C), 1458, 1380, 1054cm⁻¹;¹HNMR (500MHz, pyridin - d₅ ) δ0.86 (3H, s, Me), 0.95 ( 3H, s, Me), 1.01 ( 9H, s, 3 ×Me ), 1.32 ( 3H, s, Me), 1.39 ( 3H, s, Me), 3.39( 1H, dd, J=4.3 and 11.8Hz, H - 3α ), 3.68( 1H, d, J=10.5Hz, H - 28a ), 4.43 (1H, d, J=10.5Hz, H - 28b ), 4.68 ( 1H, m, H - 16α ), 5.04 ( 1H, d, J=7.8Hz, H - 1 of gluconic acid), 5.26 (1H, brs, H - 12 ); ¹³CNMR ( 125MHz, pyridin - d₅ ), See table 1 and 2; FAB MSm/z 657[M+Na]⁺.

### Compound B:

Amorphous; mp192 - 195°C; [α]₂₀^{D}+27.2° ( c 0.15, MeOH ); IRνₘₐₓ3444 ( OH ), 1724, 1700, 1635 ( C=C ), 1457, 1388, 1280, 1074, 720cm⁻¹; ¹HNMR ( 500MHz, pyridin) δ0.98 (3H, s, Me), 1.01 ( 3H, s, Me), 1.02(9H, s, 3 ×Me ), 1.07(3H, s, Me), 1.30 ( 3H, s, Me), 1.34 ( 3H, s, Me ), 1.36 ( 3H, s, Me), 3.40 ( 1H, dd, J=4.5 and 12.0Hz, H - 3α ), 3.70 ( 1H, d, J=10.2Hz, H-28a), 4.42 ( 1H, d, J=10.2Hz, H-28b), 4.70( 1H, m, H - 16α ), 5.10( 1H, d, J=7.8Hz, H-1 of gluconic acid), 5.70 ( 1H, dd, J=4.7 and 12.3Hz, H - 21α ), 7.47 (3H, overlap, H - 3', - 4' and - 5' ), 8.25(2H, dd, J=1.4 and 4.8Hz, H - 2' and - 6' ); ¹³CNMR ( 125MHz, pyridin - d₅ ), See table 1 and 2 ; FAB MSm/z 777[M+Na]⁺.

**Table 1:**

| ¹³CNMR data of glucoside liquid of compound A and B | | |
|---|---|---|
| Carbon atom | Compound A | Compound B |
| 1 | 38.8 | 38.8 |
| 2 | 26.6 | 26.6 |
| 3 | 89.0 | 89.0 |
| 4 | 39.5 | 39.6 |
| 5 | 55.7 | 55.7 |
| 6 | 18.4 | 18.4 |
| 7 | 32.9 | 33.0 |
| 8 | 40.1 | 40.1 |
| 9 | 47.1 | 47.1 |
| 10 | 36.7 | 36.7 |
| 11 | 23.8 | 23.9 |
| 12 | 122.6 | 123.1 |
| 13 | 143.9 | 142.6 |
| 14 | 43.8 | 43.7 |
| 15 | 36.7 | 36.8 |
| 16 | 66.6 | 66.4 |
| 17 | 41.1 | 43.8 |
| 18 | 44.4 | 44.2 |
| 19 | 47.1 | 47.2 |
| 20 | 31.1 | 36.0 |
| 21 | 34.3 | 75.6 |
| 22 | 26.2 | 33.3 |
| 23 | 28.2 | 28.2 |
| 24 | 16.9 | 16.9 |
| 25 | 15.7 | 15.7 |
| 26 | 17.0 | 17.0 |
| 27 | 27.2 | 27.0 |
| 28 | 68.9 | 66.8 |
| 29 | 33.4 | 29.2 |
| 30 | 24.1 | 18.8 |
| Acyl 1' | | 131.6 |
| Acyl 2' | | 129.9 |
| Acyl 3' | | 128.9 |
| Acyl 4' | | 133.2 |
| Acyl 5' | | 128.9 |
| Acyl 6' | | 129.9 |
| Acyl 7' | | 166.3 |

**Table 2:**

| ¹³CNMR data of saccharic part compound A and B | | |
|---|---|---|
| Carbon atom of C - 3 | Compound A | Compound B |
| Glutamic acid 1 | 107.3 | 107.3 |
| Glutamic acid 2 | 75.6 | 75.6 |
| Glutamic acid 3 | 78.2 | 78.2 |
| Glutamic acid 4 | 73.5 | 73.6 |
| Glutamic acid 5 | 77.8 | 77.7 |
| Glutamic acid 6 | 173.1 | 173.3 |

### Example 2:

Preparation of Compound C (formula II Gymnemic Acid derivative with R₃ as H and R₂ as follow group), 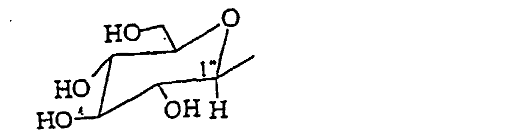 compound D (formula II Gymmemic Acid derivative with R₃ as follows and R₂ as H), 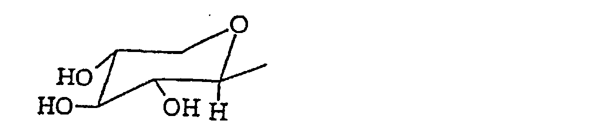 compound E(formula II Gymnemic Acid derivative with R₃ as follow),
R₃ is 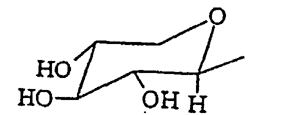 R₂ is 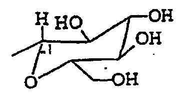 R₂ as follow and compound F(formula II Gymnemic Acid derivative with R₃ as H and R₂ as follow)

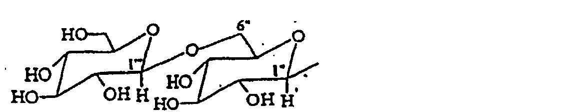

1000g raw powder of Gymnema cane leaves were refluxed for 3 times with 75% ethanol. 6.0L solvents were used, 2 hours for each time. The extract mixtures were combined together and distilled under reduced pressure till there was no ethanol, the condensed mixture was extracted with 0.5L cyclohexane and butane for 3 times. All the n-butane extract mixtures were gathered and distilled under reduced pressure to obtain 72.0g dry extract product. 36.0g dry extract substance was taken and added into 60g 60-100 mesh rough silica gel, the mixture was vaporized to dryness on a water pan, 400g 200-400 mu silica gel H used as thin-layer separation were loaded into column in a wet method , then treated sample was added undergoing column separation with elute as 90:10-50:50 chloroform-methanol mixtures ,130mg compound C, 115mg compound D , 160mg compound E and 195mg compound F were obtained respectively.

### The physics and chemistry data of compound C were shown as follows:

Amorphous powder; mp206 - 209°C; [α]₂₀^{D} - 16.0° ( c 0.11, MeOH ); IRνₘₐₓ3424 ( OH ), 1735 ( COOR), 1636 ( C=C), 1457, 1034cm⁻¹; ¹HNMR (400MHz, pyridin - d₅) δ0.82 (3H, s, Me), 0.87 (3H, s, Me), 0.91 (3H, s, Me ), 0.97 ( 3H, s, Me), 1.07 ( 3H, s, Me ), 1.20 ( 3H, s, Me), 1.23 ( 3H, s, Me), 3.17 ( 1H, dd, J=3.5 and10.2Hz, H - 18 ), 3.30( 1H, d, J=3.9 and 11.7Hz, H - 3α), 5.37( 1H, brs, H - 12), ¹³CNMR (100MHz, pyridin - d₅), See table 3 and 4; FAB MSm/z 943[M+H]⁺.

### The physics and chemistry data of compound D were shown as follows:

Amorphous powder; mp202 - 204°C; [α]₂₀^{D} - 3.2° ( c 0.15, MeOH ); IRνₘₐₓ3410( OH ), 1710(COOR), 1638(C=C), 1458, 1036cm⁻¹; ¹HNMR (400MHz, pyridin - d₅ ) δ0.87 (3H, s, Me), 0.91 ( 3H, s, Me), 0.96 (3H, s, Me), 1.02 ( 3H, s, Me), 1.10(3H, s, Me), 1.24(3H, s, Me), 1.29 (3H, s, Me), 3.30 ( 1H, dd, J=4.5 and 11.5Hz, H - 3α), 5.38 ( 1H, brs, H - 12 ), ¹³CNMR( 100MHz, pyridin - d₅ ), See table 3 and 4; FAB MSm/z 935[M+Na]⁺.

### The physics and chemistry data of compound E were shown as follows:

Amorphous powder; mp212 - 215°C; [α]₂₀^{D} - 9.6° ( c 0.20, MeOH); IRνₘₐₓ3414 (OH), 1740 (COOR), 1636 (C=C), 1460, 1364, 1044, 896cm⁻¹; ¹HNMR ( 500MHz, pyridin - d₅) δ0.85 ( 3H, s, Me), 0.90 ( 3H, s, Me), 0.94 ( 3H, s, Me), 1.00(3H, s, Me), 3.19 ( 1H, dd, J=4.0 and 13.7Hz, H - 18), 3.32(1H, d, J=4.4 and 11.7Hz, H - 3α), 5.40 ( 1H, brs, H - 12 ), ¹³CNMR ( 100MHz, pyridin - d₅ ), See table 3 and 4; FAB MSm/z 943[M+Na]⁺.

### The physics and chemistry data of compound F were shown as follows:

Amorphous powder; mp209 - 211 °C ; [α]₂₀^{D} - 12.1° ( c 0.12, MeOH ); IRνₘₐₓ3424 (OH), 1734 ( COOR ), 1636 ( C=C ), 1458, 1047cm⁻¹; ¹HNMR (400MHz, pyridin - d₅ ) δ0.87 (3H, s, Me), 0.90 (3H, s, Me), 0.92 ( 3H, s, Me), 1.00 ( 3H, s, Me), 1.09(3H, s, Me), 1.22(3H, s, Me), 1.26 ( 3H, s, Me), 3.20 ( 1H, dd, J=3.5 and 13.6Hz, H - 18 ), 3.33 ( 1H, d, J=4.4 and 11.5Hz, H - 3α), 5.39(1H, brs, H - 12), ¹³CNMR ( 100MHz, pyridin - d₅ ), See table 3 and 4; FAB MSm/z 1127[M+H]⁺.

**Table3:**

| ¹³CNMR data of glucoside ligand of compound C-F | | | | |
|---|---|---|---|---|
| Carbon atom | Compound C | Compound D | Compound E | Compound F |
| 1 | 38.8 | 38.7 | 38.7 | 38.7 |
| 2 | 26.6 | 26.7 | 26.7 | 26.7 |
| 3 | 88.9 | 89.0 | 89.0 | 89.0 |
| 4 | 39.4 | 39.5 | 39.5 | 39.5 |
| 5 | 55.7 | 55.8 | 55.8 | 55.8 |
| 6 | 18.4 | 18.3 | 18.5 | 18.5 |
| 7 | 33.0 | 33.1 | 33.1 | 33.1 |
| 8 | 39.8 | 39.9 | 39.9 | 39.9 |
| 9 | 47.9 | 48.0 | 48.0 | 48.0 |
| 10 | 36.9 | 37.0 | 37.0 | 37.0 |
| 11 | 23.7 | 23.7 | 23.8 | 23.7 |
| 12 | 122.9 | 122.8 | 123.0 | 122.9 |
| 13 | 144.0 | 144.4 | 144.0 | 144.1 |
| 14 | 42.0 | 42.1 | 42.1 | 42.1 |
| 15 | 28.2 | 28.2 | 28.2 | 28.2 |
| 16 | 23.3 | 23.4 | 23.4 | 23.4 |
| 17 | 46.9 | 46.5 | 47.0 | 47.0 |
| 18 | 41.6 | 41.9 | 41.7 | 41.7 |
| 19 | 46.2 | 46.1 | 46.2 | 46.3 |
| 20 | 30.7 | 30.9 | 30.8 | 30.8 |
| 21 | 33.9 | 34.4 | 34.0 | 34.0 |
| 22 | 32.5 | 33.1 | 32.5 | 32.5 |
| 23 | 28.1 | 28.2 | 28.2 | 28.3 |
| 24 | 17.0 | 17.0 | 17.0 | 17.0 |
| 25 | 15.5 | 15.8 | 15.6 | 15.6 |
| 26 | 17.4 | 17.3 | 17.5 | 17.5 |
| 27 | 26.0 | 26.1 | 26.1 | 26.1 |
| 28 | 176.4 | 180.2 | 176.5 | 176.5 |
| 29 | 33.1 | 33.2 | 33.2 | 33.2 |
| 30 | 23.6 | 23.7 | 23.7 | 23.7 |

**Table 4:**

| ¹³CNMR data of saccharic part of compound C-F | | | | |
|---|---|---|---|---|
| C - 3 | Compound C | Compound D | Compound E | Compound F |
| Glc1 | 106.9 | 107.0 | 107.0 | 106.9 |
| Glc2 | 75.1 | 75.0 | 75.0 | 75.2 |
| Glc3 | 78.4 | 78.3 | 78.3 | 78.4 |
| Glc4 | 71.6 | 71.5 | 71.5 | 71.5 |
| Glc5 | 77.0 | 77.0 | 77.0 | 77.0 |
| Glc6 | 70.4 | 70.4 | 70.4 | 70.5 |
| Glc'1 | 105.4 | 105.4 | 105.4 | 105.4 |
| Glc'2 | 75.5 | 75.6 | 75.6 | 75.6 |
| Glc'3 | 78.5 | 78.5 | 78.5 | 78.6 |
| Glc'4 | 71.7 | 71.6 | 71.6 | 71.7 |
| Glc'5 | 78.4 | 76.9 | 76.9 | 78.5 |
| Glc'6 | 62.7 | 69.8 | 69.8 | 62.6 |
| Xyl1 | | 106.0 | 106.0 | |
| Xyl2 | | 74.9 | 74.9 | |
| Xyl3 | | 78.0 | 78.1 | |
| Xyl4 | | 71.1 | 71.1 | |
| Xyl5 | | 67.0 | 67.1 | |

| C-28 | | | | |
|---|---|---|---|---|
| Glc"1 | 95.7 | | 95.8 | 95.7 |
| Glc"2 | 74.1 | | 74.1 | 73.9 |
| Glc"3 | 78.8 | | 78.9 | 78.7 |
| Glc"4 | 71.0 | | 71.1 | 70.9 |
| Glc"5 | 79.3 | | 79.3 | 78.0 |
| Glc"6 | 62.1 | | 62.2 | 69.3 |
| Glc'''1 | | | | 105.3 |
| Glc"'2 | | | | 75.2 |
| Glc'''3 | | | | 78.5 |
| Glc'''4 | | | | 71.7 |
| Glc'''5 | | | | 78.4 |
| Glc'''6 | | | | 62.7 |

### Biological active experiments

### Experiment example 1

Effect of compound B on the increasing blood sugar in rats caused by sucrose

Female SD rats fasted for 24 hours were divided into several groups randomly. Test groups are given 50,100,200mg/kg compound B, the positive-control group was given 100mg/kg phenformin, The normal group, control group and blank group were given the same amount of water, the given medicine volume was 10mg/kg, after 30 minutes each group was given saccharose 1/kg(5ml/kg) except normal group, and blood was extracted from the eyes of rats after 30, 60 and 120 minutes respectively, the content of glucose in the serum was measured.

The result was, after the rats were given saccharose for 30 ,60 minutes, the value of blood sugar increased apparently. The compound B 200mg/kg and phenformin 100mg/kg within 30 minutes can both reduce the increased value of blood sugar remarkably, and the strength of the two compounds was similar. Results see table 5.

**Table 5:**

| The effect of compound B on the increasing blood sugar in rats caused by sucrose | | | | |
|---|---|---|---|---|
| **(X±SD, n=10)** | | | | |
| group | dose (mg/kg) | Value of blood sugar (mmol/L) | | |
| | | 30minutes | 60 minutes | 120 minutes |
| Normal group | | 3.56±0.64 | 4.12±0.72 | 3.76±0.69 |
| control group | | 6.58±0.87^{ΔΔ} | 5.93±1.27^{ΔΔ} | 4.54±1.37 |
| compound B | 50 | 6.03±0.86 | 6.42±0.78 | 4.26±1.03 |
| | 100 | 5.12±1.29** | 5.77±1.09 | 4.53±0.94 |
| | 200 | 4.43±0.72** | 4.73±0.83** | 4.07±0.70 |
| phenformin | 100 | 4.24±0.87** | 4.74±0.90* | 4.79±1.03 |

| | | | | |
|---|---|---|---|---|
| ^{ΔΔ}P<0.01, compared with normal group; | | | | |
| *P<0.05, | | | | |
| *P<0.01, compared with control group. | | | | |

### Experiment example 2

The effect of compound B on the contents of TG, cholesterol in serum of hyperlipidemia rats

Male SD rats with the weight of 130-170g, normal group was given common food, other groups were given food having high lipid content (1%cholesterol, 10%lard, 0.3% cholic acid, 0.2% methylthio imidazole and 88.5% common forage, made into block.). In sequentially 14 days, rats fasted 12 hours were measures by reagent box method to obtain the contents of TG and cholesterol in serum. Then they were divided according to the value of blood grease into different group. The experimental group was given 50, 100. 200mg/kg compound B, the positive-control group was given clofibrate 100mg/kg, the confrol group was given water, the given medicine volume was 10ml/kg, for 10 days, each group was still given high fat forage for 5 days before given medicine, common forage was given in the later 5 days, the rats were fasted for 11 hours before being given the final administration and blood of rat were extracted to obtain the content of TG and cholesterol in serum 1 hour after being given medicine.

The results show that 10 days after rats were given forage having high grease, the contents of TG and cholesterol increased apparently, compound B 50, 100, 200mg/kg and clofibrate 100mg/kg can both reduced the contents of TG and cholesterol in blood serum of hyperlipidemia rats, compound B 200mg/kg have the same effect as 100mg/kg clofibrate in reducing hyperlipidemia, see table 6

**Table 6:**

| The effect of compound B on the contents of blood lipid in hyperlipidemia rats ( X±SD, n=9 - 10 ) | | | | | |
|---|---|---|---|---|---|
| group | dose ( mg/kg ) | TG ( mmol/L ) | | Total cholesterol ( mmol/L ) | |
| | | Before administration | After administration | Before administration | After administration |
| Normal group | | 1.02±0.22 | 1.04±0.15 | 2.43±0.41 | 1.99±0.47 |
| control group | | 2.64±0.82 | 3.04±0.93 | 4.10±0.51^{ΔΔ} | 4.77±0.63^{ΔΔ} |
| compound B | 50 | 2.72±0.61 | 2.41±0.44 | 4.29±0.60 | 3.92±0.58** |
| | 100 | 2.54±0.90 | 1.75±0.53** | 4.02±0.59 | 2.94±0.66** |
| | 200 | 2.72±0.76 | 1.37±0.40** | 4.18±0.61 | 2.31±0.74** |
| clofibrate | 100 | 2.51±0.77 | 2.72±0.74 | 4.33±0.51 | 2.15±0.76** |

| | | | | | |
|---|---|---|---|---|---|
| ^{ΔΔ}P<0.01, compared with normal group; | | | | | |
| **P<0.01, compared with control group | | | | | |

### Experiment example 3

Effect of compound B on blood platelet aggregation in rabbits invitro.

Taken blood from rabbit heart with puncture, 3.8 % potassium citrate for articoagulation (1:9), centrifuge 15 minute in 1000rpm, take upper layer as rich blood platelet plasma (prp), And then centrifuge 10 minutes with 4000rpm, take supernatant as poor blood platelet plasma (ppp). Transfer PPP200ul to nephelotube, and add into different concentration physiological brine solution 10ul of the compound B, final concentration is respectively 250,500,1000µg/ml, add physiological brine 10µl of aspirin to positive control tube ,put it into measuring cell after warming for 2 minutes in 37°C, and add into physiological brine solution10ul of ADP sodium salt with stirring, final concentration is 1.0×10⁵M. Observe the maximal aggregation ratio on PAM-1 type of blood platelet instrument within 3 minutes.

The result shows that the compound B 500,1000ug/ml and aspirin 250ug/ml obviously inhibit blood platelet from aggregating.

**Table 7:**

| Effect of compound B on blood platelet aggregation in rabbit (X± SD, n=8) | | | |
|---|---|---|---|
| Group | Final concentration | Maximal aggregation ratio (%) | Inhibition ratio |
| control group | | 47.9±5.2 | |
| compound B | 250 | 43.6±7.0 | 9.0 |
| | 500 | 35.9±4.5∗∗ | 25.1 |
| | 1000 | 27.8±4.8∗∗ | 42.0 |
| Aspirin | 250 | 23.7±6.0∗∗ | 50.3 |

| | | | |
|---|---|---|---|
| ∗∗P<0.01,compared with control group | | | |

### Experiment example 4

Effect of compound F on blood sugar elevation in rat.

Male Kun Ming strain mice are divided into randomly experiment groups, they respectively take orally the compound F 50,100,2000mg/kg, positive control group respectively take orally glybenclamide 50mg/kg, blank control group and normal control group take orally the same distilled water, the volume of medicine given is 20ml/kg, lasting 7 days. They are forbidden to give feedstuff 10 hours before the last time of administration. Each group is given dextrose solution 2.5g/kg (10ml/kg) except of normal control group. Before and after 30 minute of administration of dextrose, pick blood 100ul from eyepit, measure the content of dextrose in serum according to the way of dextrose oxygenation enzyme.

Result, after mice take orally dextrose 30 minutes , blood sugar obviously rise, Both the compound F 100,200mg/kg and 50mg/kg obviously inhibit blood sugar in mice from rising. The function of the compound B 200mg/kg and glybenclamide 500mg/kg lowering blood sugar is similar, which may be seen in table 8.

**Table 8**

| Group | | Dose (mg/kg) | Value of blood sugar |
|---|---|---|---|
| | | 0 minute | 30 minutes |
| Normal group | | 6.20±1.01 | 6.64±1.04 |
| control Group | | 6.55±1.16 | 13.94±3.22 |
| compound B | 50 | 6.79±1.16 | 12.01±1.88 |
| | 100 | 6.09±1.34 | 9.59±2.25** |
| | 200 | 6.42±0.99 | 9.16±1.08** |
| glybenclamide | 50 | 4.48±0.83** | 8.18±1.72** |

| | | | |
|---|---|---|---|
| P<0.01, compared with normal group: **p<0.01,compared with control group | | | |

### Experiment example 5

Effect of compound F on the content of triglycerides and cholesterol in the serum of hyperlipidemia rat.

Male SD rat with weight of 130-170g. The normal group is given general feedstuff, and the other groups are given high-fat (1% cholesterol, 10% pig oil, 0.3% clolic acid ,0.2 % methylthio imidazole and 88.5 % normal feedstuff are made stuff by oneself ).After the feedstuff is in run for 14 days and the mice rat is forbidden to eat for 12 hours, measure the content of triglycerides and cholesterol in rat's serum. And then the rat are grouped randomly according to blood lipid value .The experiment group is given to this compound F 50,100,200mg/kg., the ositive-control group is given orally to clofibrate of 100mg/kg, the control group is given distilled water. The volumn of administration is given 10ml/kg, lasting 10 days. Each group is given high-fat feedstuff in the former 5days of giving drugs, and then general feedstuff in the next 5days.Fasting of 11 hours is conducted before the last time of giving drugs. After giving drugs for 1 hour, take blood and measure the content of ester and cholesterol in the blood serum..

### Result

The content of TG and cholesterol in the blood serum of rat elevates obviously after given high-fat feedstuff for 10 days.50mg/kg, 100mg/kg, 200mg/kg of compound Fand 200mg/kg clofibrate make the level of triglycerides and cholesterol in blood serum of rat with high -fat blood diseases lower. The action 200mg/kg of the compound F is the similar as to that of 100mg/kg of clofibrate in the function of lowering blood fat.(table 9)

**Table 9:**

| the effect of compound B on the content of blood fat of rat with high-fat blood disease. (X±SD, n=9-10) | | | | | |
|---|---|---|---|---|---|
| group | dose (mg/kg) | triglycerides (mmol/L) | | Total cholesterin (mmol/L) | |
| | | Before administration | After administration | Before administration | After administration |
| Normal group | | 1.02±0.22 | 1.04±0.15 | 2.43±0.41 | 1.99±0.47 |
| Control group | | 2.64±0.82 | 3.04±0.93 | 4.10±0.51^{ΔΔ} | 4.77±0.63^{ΔΔ} |
| compound B | 50 | 2.72±0.61 | 2.41±0.44 | 4.29±0.60 | 3.92±0.58** |
| | 100 | 2.54±0.90 | 1.75±0.53** | 4.02±0.59 | 2.94±0.66** |
| | 200 | 2.72±0.76 | 1.37±0.40** | 4.18±0.61 | 2.31±0.74** |
| clofibrate | 100 | 2.51±0.77 | 2.72±0.74 | 4.33±0.51 | 2.15±0.76** |

| | | | | | |
|---|---|---|---|---|---|
| ^{ΔΔ}P<0.01, (compared with normalgroup); | | | | | |
| **P<0.01(compared with control group) | | | | | |

### Experimental example 6 effect of compound B affects on blood platelet aggregation in rabbit.

Take blood from rabbit heart by puncturing,3.8% of potassium citrate for anticoagulation (1:9), centrifuguging 15 minute in 1000rpm,take the upper layer as blood platelet rich plasma(prp), And then centrifuguga 10 minute in 4000rpm,take supernatant as blood platelet poor plasma(ppp).the final concentration of compound B is revetively 250,500,1000 µ g/ml, final concentration is respectively250,500,1000ug/ml, add 10ul of physical brine of aspirin to the positive-control tube to final concentration of 250 µ g/ml, add 10ul of physical brine to control tube to the final concentration of 250 µ g/ml .Observe the maximal aggregation ratio on PAM-1type instrument of blood platelet aggregation within 3 minute.

The result shows that 500,1000µg/ml of the compound F and aspirin 250µg/ml obviously inhibit the aggregation of blood platelet.

**Table 10:**

| the effect of the compound F on aggregation of rabbit's blood platelets in vitro. | | | |
|---|---|---|---|
| group | Final concentration (µg/ml) | Maximal aggregation rate (%) | Inhibition rate (%) |
| control | | 47.9±5.2 | |
| compound F | 250 | 43.6±7.0 | 9.0 |
| | 500 | 35.9±4.5** | 25.1 |
| | 1000 | 27.8±4.8** | 42.0 |
| aspirin | 250 | 23.7±6.0** | 50.3 |

| | | | |
|---|---|---|---|
| **P<0.01(compared with the control) | | | |

### Experimental example 7

Effect of compound B on blood sugar in normal mice.

Male Kun Ming strain mice are divided into randomly experiment groups, they respectively take orally the compound B 50,100,2000mg/kg, positive control group respectively take orally tolbutol 100mg/kg, blank control group takes orally the same distilled water, the volume of medicine given is 20ml/kg, lasting 14 days. They are administrated to test drug (provided that they are pre-forbidden to give food 5 hrs before administration) after the days 1, 3, 7, 14 of administration. After 3hrs of administration, pick blood 10ul from eyepit, measure the content of dextrose in serum according to the way of reagent box.

Result shows that compound B 50, 100, 200mg/kg for continucly administration 14 days has no obvious effect on blood sgar of normal onice, but tolbutol starting from day 3 of administration show obvious effect for lowering the blood sugar of normal mice. Result is also seen in table 11.

**Table 11,**

| Effect of compound B on blood sugar in normal mice. **( X±SD, n=10 )** | | | | | |
|---|---|---|---|---|---|
| Group | Dose (mg/kg) | Value of blood sugar | | | |
| | | 1 | 3 | 7 | 14 (day) |
| control Group | | 5.21±1.10 | 7.10±1.30 | 8.56±0.74 | 7.52±1.29 |
| compound B | 50 | 5.84±0.94 | 7.56±0.92 | 8.51±1.06 | 8.27±0.66 |
| | 100 | 6.48±1.28 | 7.73±2.26 | 8.71±0.97 | 7.45±1.59 |
| | 200 | 6.41±1.04 | 6.28±1.19 | 8.46±0.88 | 7.86±1.56 |
| tolbutol | 100 | 6.48±1.18 | 5.22±0.80** | 6.62±0.96 | 5.75±1.02 |

| | | | | | |
|---|---|---|---|---|---|
| P<0.01, compared with normal group: **p<0.01, compared with control group | | | | | |

## Claims

1. Gymnemic acid derivatives of general formula I or general formula II 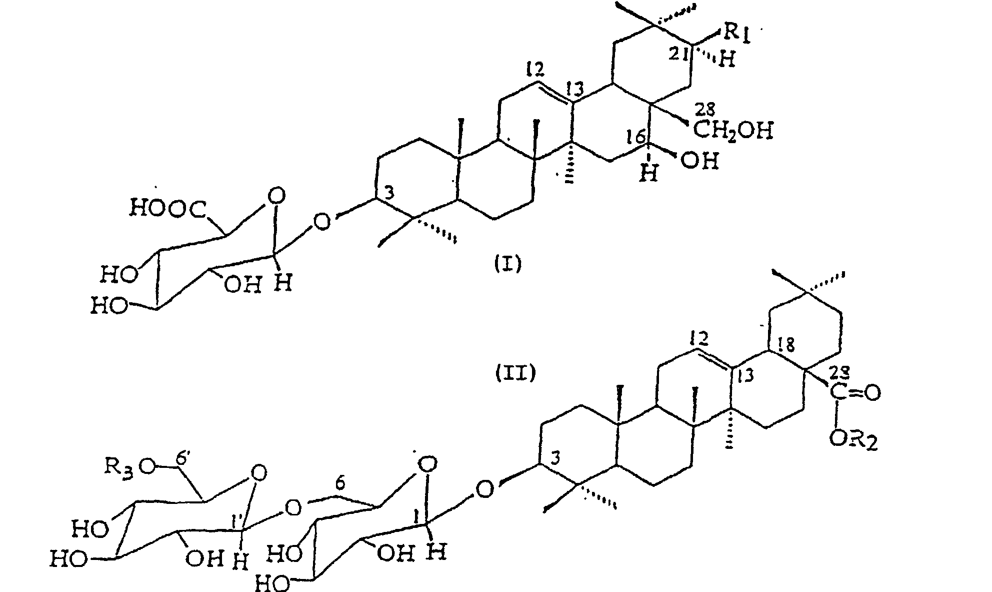 wherein R₁ is H or the radical of formula III 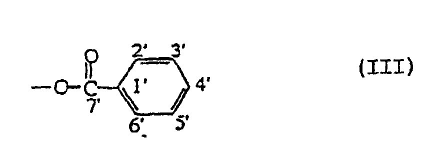 either R₃ is H and R₂ is a radical of formula IV or V 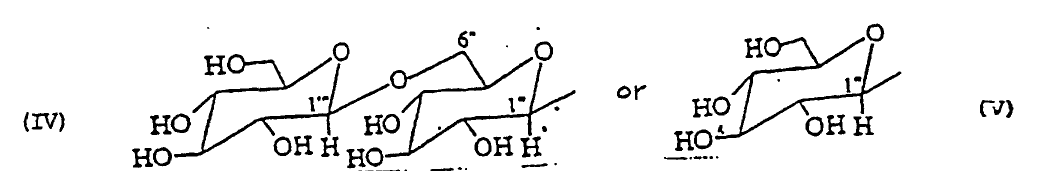 or R₃ is a radical of formula VI 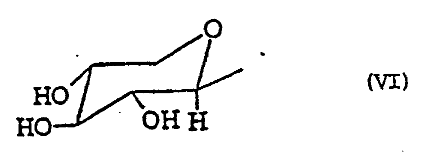 and R₂ is H or a radical of formula VII 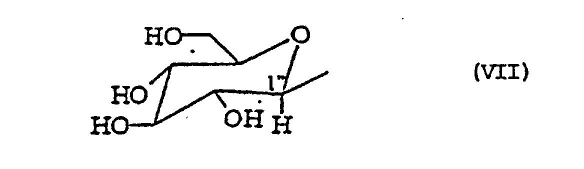 or a pharmaceutical base addition salt thereof.

2. The gymnemic acid derivatives of claim 1, wherein R₁ in formula I is hydrogen.

3. The gymnemic acid derivatives of claim 1, wherein R₁ in formula I is a radical of formula III.

4. The gymnemic acid derivatives of claim 1, wherein R₃ in formula II is hydrogen and R₂ is a radical of formula IV.

5. The gymnemic acid derivatives of claim 1, wherein R₃ in formula II is hydrogen and R₂ is a radical of formula V.

6. The gymnemic acid derivatives of claim 1, wherein R₂ in formula II is hydrogen and R₃ is a radical of formula VI.

7. The gymnemic acid derivatives of claim 1, wherein R₃ in formula II is a radical of formula VI and R₂ is a radical of formula VII.

8. A pharmaceutical composition which contains at least one kind of gymnemic acid derivatives of formula I and/or II of claim 1 or a pharmaceutical base addition salt thereof as active ingredient, pharmaceutical carrier and excipient.

9. A pharmaceutical composition for the prevention or treatment of the diseases associated with hyperglycemia, hyperlipidemia and/or platelets aggregation, which contains at least one kind of gymnemic acid derivatives of formula I and/or II of claim 1 or a pharmaceutical base addition salt thereof as a active ingredient, pharmaceutical carrier and excipient.

10. The composition of claim 8 or 9, which contains gymnemic acid derivatives of formula I wherein R₁ is H (compound A), formula I wherein R₁ is a radical of formula III (compound B), formula II wherein R₃ is H and R₂ is a radical of formula VII (compound C), formula II wherein R₂ is H and R₃ is a radical of formula VI (compound D), of formula II wherein R₂ is a radical of formula V and R₃ is a radical of formula VI (compound E) and/or of formula II wherein R₂ is H and R₂ is a radical of formula IV (compound F), wherein based on the weight of the composition, the content of compounds A, B, C, D, E and F is 1.25-2.10 % compound A, 0.89-1.50% compound B, 2.40-3.80% compound C, 2.10-3.40% compound D, 2.74-4.60% compound E and/or 3.24-5.40% % compound F.

11. A extract of Gymnema sylvestre.R.Br which contains 12.5-40 wt.-% gymnemic acid derivatives of formula I and formula II of claim 1.

12. Use of gymnemic acid derivatives of formula I and II of claim 1, or the extract containing gymnemic acid derivatives of formula I and II, for the manufacture of a medicament for the prevention or treatment of the diseases and conditions associated with hyperglycemia, hyperlipidemia and/or platelets aggregation.

13. A method for the preparation of gymnemic acid derivatives of formula I and II of claim 1 or pharmaceutical base addition salts thereof, which includes the following steps:
a) extracting the plant Gymnema cane with ethanol under reflux and then concentrating;
b) extraction the concentrated liquid from step a) with cyclohexane, then extracting with n-butanol, concentrating to dryness under reduced pressure, and then obtaining a residue;
c) subjecting the residue from step b) to silica column chromatography with elution agent chloroform: methanol=90:10-50:5 or 90:10-60:40 to obtain gymnemic acid derivative of formula I and residue;
d) subjecting the residue from step c) to C₁₈ column chromatography with elution agent methanol water (20/80-40/60) to obtain gymnemic acid derivatives of formula II; and, optionally
e) converting the obtained gymnemic acid derivatives of formula I or II into a pharmaceutical base addition salt with an inorganic or organic base.

## Patentansprüche

1. Gymnemasäurederivate der allgemeinen Formel I oder der allgemeinen Formel II 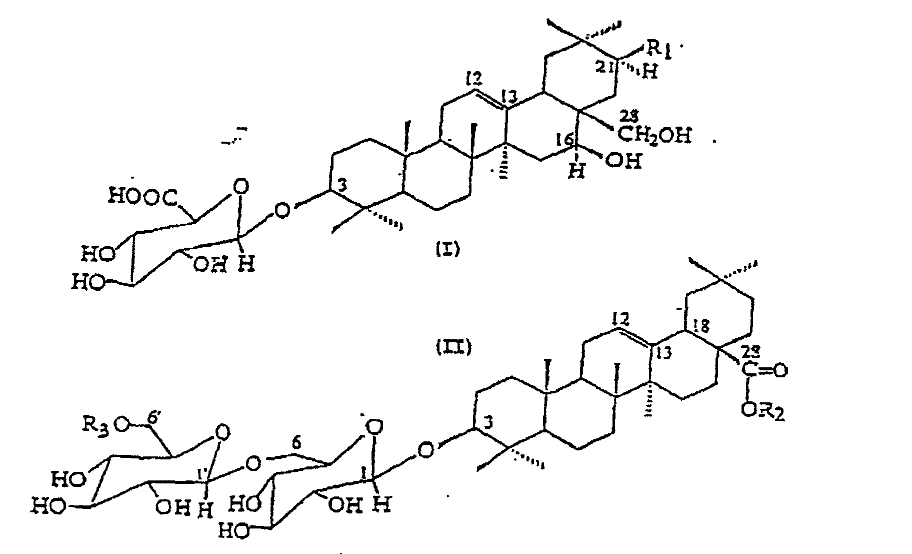 in der R₁ H oder den Rest der Formel III 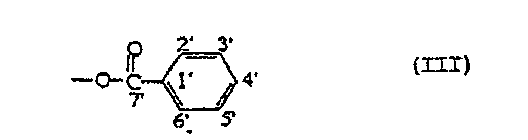 bedeutet und entweder R₃ H bedeutet und R₂ einen Rest der Formel IV oder V 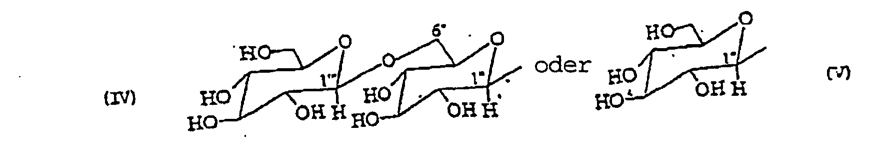 bedeutet oder R₃ einen Rest der Formel VI 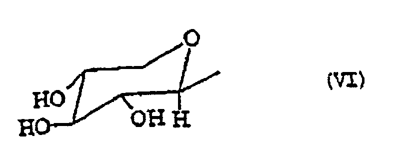 bedeutet und R₂ H oder einen Rest der Formel VII 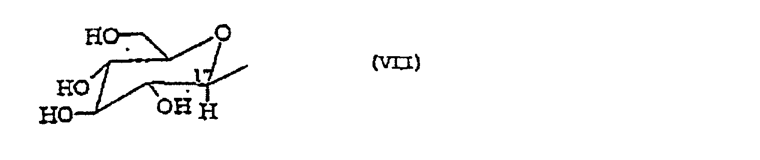 bedeutet,
bzw. eines ihrer pharmazeutischen Basenadditionssalze.

2. Gymnemasäurederivate nach Anspruch 1, wobei R₁ in Formel I Wasserstoff bedeutet.

3. Gymnemasäurederivate nach Anspruch 1, wobei R₁ in Formel I einen Rest der Formel III bedeutet.

4. Gymnemasäurederivate nach Anspruch 1, wobei R₃ in Formel II Wasserstoff bedeutet und R₂ einen Rest der Formel IV bedeutet.

5. Gymnemasäurederivate nach Anspruch 1, wobei R₃ in Formel II Wasserstoff bedeutet und R₂ einen Rest der Formel V bedeutet.

6. Gymnemasäurederivate nach Anspruch 1, wobei R₂ in Formel II Wasserstoff bedeutet und R₃ einen Rest der Formel VI bedeutet.

7. Gymnemasäurederivate nach Anspruch 1, wobei R₃ in Formel II einen Rest der Formel VI bedeutet und R₂ einen Rest der Formel VII bedeutet.

8. Pharmazeutische Zusammensetzung, die mindestens eine Art von Gymnemasäurederivaten der Formel I und/oder II nach Anspruch 1 oder eines ihrer pharmazeutischen Basenadditionssalze als Wirkstoff, von pharmazeutischem Träger und von Grundstoff enthält.

9. Pharmazeutische Zusammensetzung zur Vorbeugung oder Behandlung der mit Hyperglykämie, Hyperlipidämie und/oder Thrombozytenaggregation einhergehenden Krankheiten, die mindestens eine Art von Gymnemasäurederivaten der Formel I und/oder II nach Anspruch 1 oder eines ihrer pharmazeutischen Basenadditionssalze als Wirkstoff, von pharmazeutischem Träger und von Grundstoff enthält.

10. Zusammensetzung nach Anspruch 8 oder 9, die Gymnemasäurederivate der Formel I enthält, in der R₁ H bedeutet (Verbindung A), der Formel I, in der R₁ einen Rest der Formel III bedeutet (Verbindung B), der Formel II, in der R₃ H bedeutet und R₂ einen Rest der Formel VII bedeutet (Verbindung C), der Formel II, in der R₂ H bedeutet und R₃ einen Rest der Formel VI bedeutet (Verbindung D), der Formel II, in der R₂ einen Rest der Formel V bedeutet und R₃ einen Rest der Formel VI bedeutet (Verbindung E), und/oder der Formel II, in der R₂ H bedeutet und R₂ einen Rest der Formel IV bedeutet (Verbindung F), bei der der Gehalt an den Verbindungen A, B, C, D, E und F in bezug auf das Gewicht der Zusammensetzung 1,25-2,10% Verbindung A, 0,89-1,50% Verbindung B, 2,40-3,80% Verbindung C, 2,10-3,40% Verbindung D, 2,74-4,60% Verbindung E und/oder 3,24-5,40% Verbindung F beträgt.

11. Extrakt von Gymnema sylvestre R. Br., der 12,5-40 Gew.-% Gymnemasäurederivate der Formel I und der Formel II nach Anspruch 1 enthält.

12. Verwendung von Gymnemasäurederivaten der Formel I und II nach Anspruch 1 oder extraktenthaltenden Gymnemasäurederivaten der Formel I und II zur Herstellung eines Arzneimittels zur Vorbeugung oder Behandlung der mit Hyperglykämie, Hyperlipidämie und/oder Thrombozytenaggregation einhergehenden Krankheiten und Beschwerden.

13. Verfahren zur Herstellung von Gymnemasäurederivaten der Formel I und II nach Anspruch 1 oder von deren pharmazeutischen Basenadditionssalzen mit den folgenden Schritten:
a) Extraktion der Pflanze Gymnema-Rohr mit Ethanol unter Rückfluß und anschließende Aufkonzentrierung,
b) Extraktion der konzentrierten Flüssigkeit aus Schritt a) mit Cyclohexan, anschließende Extraktion mit n-Butanol, Einengen des Extrakts zur Trockne unter verringertem Druck und Gewinnung eines Rückstandes,
c) Chromatographieren des Rückstandes aus Schritt b) an einer Silikasäule mit Chloroform:Methanol = 90:10-50:5 oder 90:10-60:40 als Elutionsmittel, wodurch man zu dem Gymnemasäurederivat der Formel I und einem Rückstand gelangt,
d) Chromatographieren des Rückstandes aus Schritt c) an einer C₁₈-Säule mit Methanol/Wasser (20/80-40/60) als Elutionsmittel, wodurch man Gymnemasäurederivate der Formel II erhält, sowie gegebenenfalls
e) Überführen der erhaltenen Gymnemasäurederivate der Formel I oder II in ein pharmazeutisches Basenadditionssalz mit einer anorganischen oder organischen Base.

## Revendications

1. Dérivés de l'acide gymnémique, de formule générale I ou de formule générale II 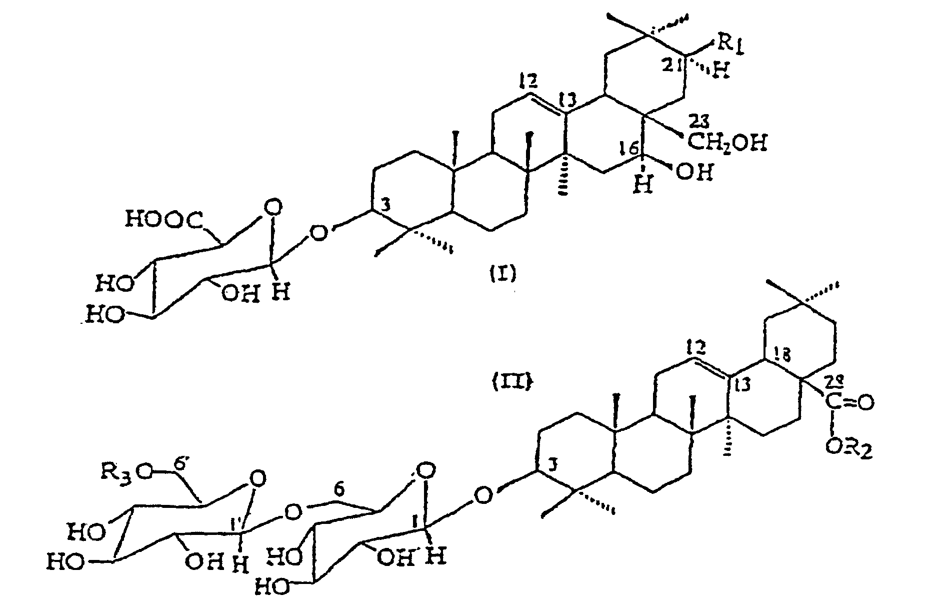 dans lesquelles R₁ est H ou le radical de formule III 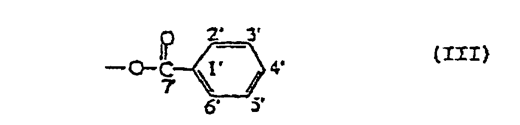 soit R₃ est H et R₂ est un radical de formule IV ou V 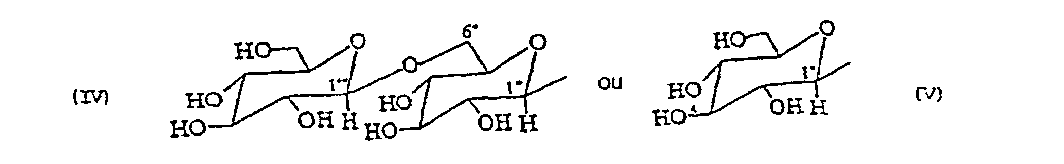 soit R₃ est un radical de formule VI 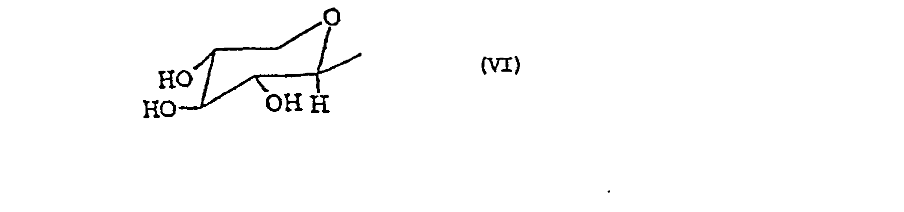 et R₂ est H ou un radical de formule VII 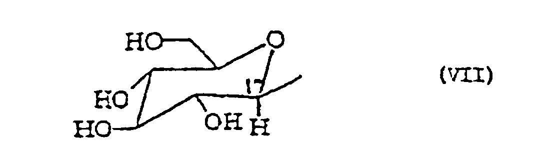 ou un sel pharmaceutique d'addition de base de ceux-ci.

2. Dérivés de l'acide gymnémique selon la revendication 1, dans lesquels R₁ dans la formule I est l'hydrogène.

3. Dérivés de l'acide gymnémique selon la revendication 1, dans lesquels R₁ dans la formule I est un radical de formule III.

4. Dérivés de l'acide gymnémique selon la revendication 1, dans lesquels R₃ dans la formule II est l'hydrogène et R₂ est un radical de formule IV.

5. Dérivés de l'acide gymnémique selon la revendication 1, dans lesquels R₃ dans la formule II est l'hydrogène et R₂ est un radical de formule V.

6. Dérivés de l'acide gymnémique selon la revendication 1, dans lesquels R₂ dans la formule II est l'hydrogène et R₃ un est un radical de formule VI.

7. Dérivés de l'acide gymnémique selon la revendication 1, dans lesquels R₃ dans la formule II est un radical de formule VI et R₂ est un radical de formule VII.

8. Composition pharmaceutique qui contient au moins un type de dérivés de l'acide gymnémique de formule I et/ou II selon la revendication 1 ou un sel pharmaceutique d'addition de base de ceux-ci, en tant qu'ingrédient actif, un véhicule et un excipient pharmaceutiques.

9. Composition pharmaceutique pour la prévention ou le traitement des maladies associées à l'hyperglycémie, à l'hyperlipidémie et/ou à l'agrégation des plaquettes, contenant au moins un type de dérivés de l'acide gymnémique de formule I et/ou II selon la revendication 1 ou un sel pharmaceutique d'addition de base de ceux-ci, en tant qu'ingrédient actif, un véhicule et un excipient pharmaceutiques.

10. Composition selon la revendication 8 ou 9, contenant des dérivés de l'acide gymnémique de formule I dans laquelle R₁ est H (composé A), de formule I dans laquelle R₁ est un radical de formule III (composé B), de formule II dans laquelle R₃ est H et R₂ est un radical de formule VII (composé C), de formule II dans laquelle R₂ est H et un R₃ est un radical de formule VI (composé D), de formule II dans laquelle R₂ est un radical de formule V et R₃ est un radical de formule VI (composé E) et/ou de formule II dans laquelle R₂ est H et R₂ est un radical de formule IV (composé F), dans laquelle, par rapport au poids de la composition, la teneur en composés A, B, C, D, E et F est de 1,25-2,10 % de composé A, 0,89-1,50 % de composé B, 2,40-3,80 % de composé C, 2,10-3,40 % de composé D, 2,74-4,60 % de composé E et/ou 3,24-5,40 % de composé F.

11. Extrait de gymnène sylvestre R.Br contenant 12,5-40 % en poids de dérivés de l'acide gymnémique de formule I et de formule II selon la revendication 1.

12. Utilisation de dérivés de l'acide gymnémique de formules I et II selon la revendication 1, ou de l'extrait contenant des dérivés de l'acide gymnémique de formules I et II, pour la fabrication d'un médicament pour la prévention ou le traitement des maladies et des états associés à l'hyperglycémie, à l'hyperlipidémie et/ou à l'agrégation des plaquettes.

13. Procédé de préparation de dérivés de l'acide gymnémique de formules I et II selon la revendication 1 ou de sels pharmaceutiques d'addition de base de ceux-ci, comprenant les étapes suivantes :
a) extraire la canne de gymnène avec de l'éthanol, au reflux, puis la concentrer ;
b) extraire le liquide concentré de l'étape a) avec du cyclohexane, puis l'extraire avec du n-butanol, le concentrer jusqu'à siccité totale à une pression réduite, puis obtenir un résidu;
c) soumettre le résidu de l'étape b) à une chromatographie sur une colonne de silice avec, en tant qu'éluant, du chloroforme:méthanol = 90:10-50:5 ou 90:10-60:40, pour obtenir un dérivé de l'acide gymnémique de formule I et un résidu ;
d) soumettre le résidu de l'étape c) à une chromatographie sur une colonne C₁₈ avec, en tant qu'éluant, du méthanol/eau (20/80-40/60), pour obtenir des dérivés de l'acide gymnémique de formule II ; et, le cas échéant,
e) transformer les dérivés obtuns de l'acide gymnémique de formule I ou II en un sel pharmaceutique d'addition de base avec une base inorganique ou organique.
